# EUROPEAN PATENT SPECIFICATION

(11) **EP 0 939 630 B1**
(45) Date of publication and mention of the grant of the patent: **30.06.2004**
(21) Application number: 97909300.2
(22) Date of filing: 15.09.1997
(51) Int. Cl.: A61K 31/35, A61P 25/02

(54) **USE OF BENZOPYRANOLS TO TREAT NEUROLOGICAL DISORDERS**
VERWENDUNG VON BENZOPYRANOLEN ZUR BEHANDLUNG VON NEUROLOGISCHEN ERKRANKUNGEN
UTILISATION DE BENZOPYRANOL DANS LE TRAITEMENT D'AFFECTIONS NEUROLOGIQUES

(30) Priority: 18.09.1996 GB 9619492
(43) Date of publication of application: 08.09.1999
(73) Proprietor: SmithKline Beecham plc, Brentford, Middlesex TW8 9GS (GB)
(72) Inventor: PARSONS, Andrew A., SmithKline Beecham Pharm., Harlowe, Essex CM19 5AW (GB); THOMPSON, Mervyn, SmithKline Beecham Pharm., Harlow, Essex CM19 5AW (GB); UPTON, Neil, SmithKline Beecham Pharm., Harlow, Essex CM19 5AW (GB); EVANS, John Morris, SmithKline Beecham Pharm., Harlow, Essex CM19 5AW (GB)
(74) Representative: West, Vivien
(86) International application number: PCT/EP1997/005168
(87) International publication number: WO 1998/011890

(56) References cited:
- WO-A-92/22293
- WO-A-95/34545
- WO-A-95/34546
- US-A- 5 510 375

## Description

This invention relates to a novel use of certain benzopyran compounds.

EP-A-0 126 311 discloses substituted benzopyran compounds having blood pressure lowering activity, including 6-acetyl-*trans*-4-(4-fluorobenzoylamino)-3,4-dihydro-2,2-dimethyl-2H-1-benzopyran-3-ol.

Also EP-A-0 376 524, EP-A-0 205 292, EP-A-0 250 077, EP-A-0 093 535, EP-A-0 150 202, EP-A-0 076 075 and WO/89/05808 (Beecham Group plc) describe certain benzopyran derivatives which possess anti-hypertensive activity.

EP-A-0 350 805 (Beiersdorf), EP-A-0 277 611, EP-A-0 277 612, EP-A-0 337 179 and EP-A-0 355 565 (Hoechst Aktiengesellschaft); EP-A-0 466 131 (Nissan Chemical Industries Ltd), EP-A-0 339 562 (Yoshitomi Pharmaceuticals), EP-A-0 415 065 (E. Merck), EP-A-0 450 415 (Squibb), EP-A-0 482 934, EP-A-0 296 975, JO-2004-791 and WO89/07103 also describe certain benzopyran derivatives which are believed to possess anti-hypertensive activity.

EP-A-0 430 621 and EP-A-0 385 584 (Beecham Group plc) describe the resolution of certain intermediates useful in the preparation of the compounds described in the above mentioned patent applications.

EP-A-0 139 992 (Beecham Group plc) describes certain benzopyran derivatives which have *cis* isomerism at position 3 and 4 which compounds are described as possessing anti-hypertensive activity.

EP-A-0 587 645, EP-A-0 673 373, EP-A-0 673 374, EP-A-0 673 248, EP-A-0 674 519, WO95/34545, WO95/34547 and WO95/34546 (SmithKline Beecham plc) describe groups of compounds possessing *inter alia* anti-convulsant activity, and which are also believed to have utility in the treatment or prevention of mania, depression and the effects associated with withdrawal from substances of abuse.

It has now been surprisingly found that compounds from the above groups have additional activity and are believed to have utility in the treatment and/or prophylaxis of degenerative diseases such as Huntingdon's chorea, schizophrenia, neurological deficits associated with AIDS, sleep disorders (including circadian rhythm disorders, insomnia and narcolepsy), tics (e.g. Giles de la Tourette's syndrome), traumatic brain injury, tinnitus, neuralgia, especially trigeminal neuralgia, neuropathic pain, dental pain, cancer pain, inappropriate neuronal activity resulting in neurodysttiesias in diseases such as diabetes, multiple sclerosis (MS) and motor neurone disease, ataxias, muscular rigidity (spasticity) and temporomandibular joint dysfunction.

Accordingly, the present invention provides the use in the manufacture of a medicament for use in the treatment and/or prophylaxis of neuralgia, especially trigeminal neuralgia, neuropathic pain, dental pain and cancer pain of a compound of formula (A) or pharmaceutically acceptable salt or solvate thereof: 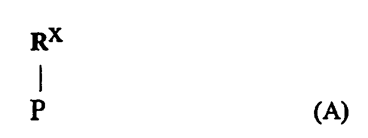 wherein:
P is a ring system 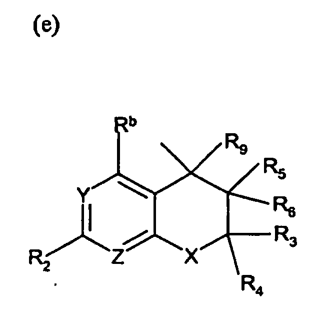 wherein:
either Y is N and R₂ is hydrogen, or Y is C-R₁; where:
   either one of R₁ and R₂ is hydrogen and the other is selected from the class of hydrogen, C₃₋₈ cycloalkyl, C₁₋₆ alkyl optionally interrupted by oxygen or substituted by hydroxy, C₁₋₆ alkoxy or substituted aminocarbonyl, C₁₋₆ alkylcarbonyl, C₁₋₆ alkoxycarbonyl, C₁₋₆ alkylcarbonyloxy, C₁₋₆ alkoxy, nitro, cyano, halo, trifluoromethyl, CF₃S, or a group CF₃-A-, where A is -CF₂-, -CO-, -CH₂-, CH(OH), SO₂, SO, CH₂-O, or CONH, or a group CF₂H-A'- where A' is oxygen, sulfur, SO, SO₂, CF₂ or CFH; trifluoromethoxy, C₁₋₆ alkylsulfinyl, perfluoro C₂₋₆ alkylsulfonyl, C₁₋₆ alkylsulfonyl, C₁₋₆ alkoxysulfinyl, C₁₋₆ alkoxysulfonyl, aryl, heteroaryl, arylcarbonyl, heteroarylcarbonyl, phosphono, arylcarbonyloxy, heteroarylcarbonyloxy, arylsulfinyl, heteroarylsulfinyl, substituted by one or two C₁₋₆ alkyl groups, or C₁₋₆ alkylsulfinylamino, C₁₋₆ alkylsulfonylamino,C₁₋₆ alkoxysulfinylamino or C₁₋₆ alkoxysulfonylamino, or ethylenyl terminally substituted by C₁₋₆ alkylcarbonyl, nitro or cyano, or -C(C₁₋₆ alkyl)NOH or -C(C₁₋₆ alkyl)NNH₂,
   or one of R₁ and R₂ is nitro, cyano or C₁₋₃ alkylcarbonyl and the other is halo, C₁₋₄ alkyl, methoxy or amino optionally substituted by one or two C₁₋₆ alkyl or by C₂₋₇ alkanoyl;
   or R₁ and R₂ together are -(CH₂)₄-; (CH₂)ₓ CO (CH₂)y where x is 0 to 3 and y is 0 to 3 with the proviso that x + y is at least 2x; or -CH = CH-CH = CH-; or form an optionally substituted triazole or oxadiazole ring, or together form a group CONR^{c}CO where R^{c} is hydrogen, C₁₋₆ alkyl, aralkyl or heteroarylalkyl;
   Z is N only when Y is C-R₁ or Z is C-R^{a} when Y is N or C-R₁; wherein R^{a} is hydrogen, halogen, nitro; C₁₋₄ alkylcarbonyl, C₁₋₄ alkyl; aryl C₁₋₄ alkyl, aryl C₁₋₄ alkenyl, heteroaryl C₁₋₄ alkyl or heteroaryl C₁₋₄ alkenyl,
   R^{b} is hydrogen, halogen, nitro; C₁₋₄ alkylcarbonyl or C₁₋₄ alkyl; and in which any aryl or heteroaryl or alkyl moiety associated with R^{a} or R^{b} are optionally substituted;
   one of R₃ and R₄ is hydrogen or C₁₋₄ alkyl and the other is C₁₋₄ alkyl, CF₃ or CH₂X^{a} where X^{a} is fluoro, chloro, bromo, iodo, C₁₋₄ alkoxy, hydroxy, C₁₋₄ alkylcarbonyloxy, -S-C₁₋₄ alkyl, nitro, amino optionally substituted by one or two C₁₋₄ alkyl groups; cyano or C₁₋₄ alkoxycarbonyl
   or R₃ and R₄ together are C₂₋₅ polymethylene optionally substituted by C₁₋₄ alkyl;
   R₅ is C₁₋₆ alkylcarbonyloxy, benzoyloxy, ONO₂, benzyloxy, phenyloxy or C₁₋₆ alkoxy and R₆ and R₉ are hydrogen or R₅ is hydroxy and R₆ and R₉ are independently hydrogen or C₁₋₂ alkyl;
   R^{x} is a 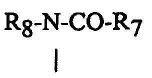 group where
   R₇ is heteroaryl or phenyl; both of which are optionally substituted one or more times independently with a group or atom selected from chloro, fluoro, bromo, iodo, nitro, amino optionally substituted once or twice by C₁₋₄ alkyl, cyano, azido, C₁₋₄ alkyl, C₁₋₄ alkoxy, trifluoromethoxy, trifluoromethyl; optionally substituted aryloxy or heteroaryloxy; C₁₋₄ alkoxy substituted by one or more halogens (excluding trifluoromethoxy); amino substituted by C₁₋₄ alkanoyl, aroyl aryl phenylsulfonyl or C₁₋₄ alkylsulfonyl; C₁₋₄ alkyl substituted by one or more halogens (excluding trifluoromethyl) or alkoxy; phenylsulfonyl C₁₋₄ alkyl sulfonyl, aminosulfonyl in which the amino group is optionally substituted by C₁₋₄ alkyl; CONH₂ in which the amino group is optionally substituted by C₁₋₄ alkyl;
   R₈ is hydrogen; C₁₋₆ alkyl, OR₁₆ or NHCOR₁₇ wherein R₁₆ is hydrogen, C₁₋₆ alkyl, formyl, C₁₋₆ alkanoyl, aroyl or aryl-C₁₋₆ alkyl and R₁₇ is hydrogen, C₁₋₆ alkyl, C₁₋₆ alkoxy, mono or di C₁₋₆ alkyl amino, amino, amino-C₁₋₆ alkyl, hydroxy-C₁₋₆alkyl, halo-C₁₋₆ alkyl, C₁₋₆ acyloxy-C₁₋₆ alkyl, C₁₋₆ alkoxycarbonyl-C₁₋₆-alkyl, aryl or heteroaryl;
   X is oxygen or NR₁₈ where R₁₈ is hydrogen or C₁₋₆ alkyl; and the R^{x} group is *cis* or *trans* to the R₅ group.

Generally the *cis* compounds of formula (A) may be prepared from the corresponding *trans* compounds, procedures for the preparation of which are generally described in EP-0126311, EP-0376524, EP-205292, EP-0250077, EP-0093535, EP-0150202, EP-0076075, WO/89/05808, EP-0350805, EP-0277611, EP-0277612, EP-0337179, EP-0339562, EP-0355565, EP-A-415 065 (E. Merck), EP-A-450 415 (Squibb) EP-0466131, EP-A-0482934, EP-A-0296975, JO-2004-791 and WO89/07103.

The *cis* compounds of formula (A) may be prepared by procedures generally described in or analogous to those described in EP-A-0139992.

The *cis* compounds of formula (A) may also be prepared according to the procedures described by G. Burrell *et al,* Tet. Letters, **31**, 3649-3652 (1990) or by the procedures described by U. Quast and E. Villhauer, Eur. J. Pharmacol, Molecular Pharmacology Section **245**,165-171 (1993).

It should be appreciated that racemates for formula (A) may be resolved or enantiomerically purified compounds of formula (A) may be prepared using procedures conventional in the art and in particular using the procedures outlined in EP-0430631 and EP-0355584.

It should also be appreciated that it is preferred that the compounds of formula (A) may be prepared in the required enantiomeric form by forming a chirally pure epoxide using catalysts and conditions generally outlined in WO91/14694 or WO93/17026 and thereafter converting the epoxides to the required compound of formula (A) using procedures outlined herein.

The *trans* compounds of formula (A) may be prepared according to the procedures outlined in PCT/GB92/01045 which procedures are incorporated herein by reference or the *trans* compounds of formula (A) may be prepared according to methods analogous to these described in the one mentioned patents.

Reference is particularly directed to EP-A-0 587 645, EP-A-0 673 373, EP-A-0 673 374, EP-A-0 673 248, EP-A-0674 519, WO95/34545, WO95/34547 and WO95/34546 for compounds suitable for use in this invention.

Preferred compounds for use in this invention are *trans*-6-acetyl-4S-(4-fluorobenzoylamino)-3,4-dihydro-2,2-dimethyl-2H-1-benzopyran-3R-ol, hereinafter Compound 1 (for preparation see Example 20 of WO 92/22293) and *cis*-6-acetyl-4S-(3-chloro-4-fluorobenzoylamino)-3,4-dihydro-2,2-dimethyl-2H-1-benzopyran-3S-ol, hereinafter Compound 2 (for preparation see Example 17 of W095/34545) and *trans*-6-acetyl-4S-(3,5-difluorobenzoylamino)-3,4-dihydro-2,2-dimethyl-2H-1-benzopyran-3R-ol, hereinafter Compound 3 (for preparation see Example 4 of W095/34545).

The above compounds may be used in therapy as pharmaceutically acceptable salts, such as hydrochlorides, and pharmaceutically acceptable solvates, such as hydrates.

The administration to the mammal may be by way of oral, parenteral, sub-lingual or transdermal administration.

An amount effective to treat the disorders hereinbefore described depends on the usual factors such as the nature and severity of the disorders being treated and the weight of the mammal. However, a unit dose will normally contain 1 to 5000 mg, suitably 1 to 500 mg, for example an amount in the range of from 2 to 400 mg such as 2, 5, 10, 20, 30, 40, 50, 100, 200, 300 and 400 mg of the active compound. Unit doses will normally be administered once or more than once per day, for example 1, 2, 3, 4, 5 or 6 times a day, more usually 1 to 4 times a day, such that the total daily dose is normally in the range, for a 70 kg adult, of 1 to 5000 mg, for example 1 to 500 mg, that is in the range of approximately 0.01 to 15 mg/kg/day, more usually 0.1 to 6 mg/kg/day, for example 1 to 6 mg/kg/day.

It is greatly preferred that the compound of formula (I) is administered in the form of a unit-dose composition, such as a unit dose oral, including sub-lingual, rectal, topical or parenteral (especially intravenous) composition.

Such compositions are prepared by admixture and are suitably adapted for oral or parenteral administration, and as such may be in the form of tablets, capsules, oral liquid preparations, powders, granules, lozenges, reconstitutable powders, injectable and infusable solutions or suspensions or suppositories. Orally administrable compositions are preferred, in particular shaped oral compositions, since they are more convenient for general use.

Tablets and capsules for oral administration are usually presented in a unit dose, and contain conventional excipients such as binding agents, fillers, diluents, tabletting agents, lubricants, disintegrants, colourants, flavourings, and wetting agents. The tablets may be coated according to well known methods in the art.

Suitable fillers for use include cellulose, mannitol, lactose and other similar agents. Suitable disintegrants include starch, polyvinylpyrrolidone and starch derivatives such as sodium starch glycollate. Suitable lubricants include, for example, magnesium stearate. Suitable pharmaceutically acceptable wetting agents include sodium lauryl sulfate.

These solid oral compositions may be prepared by conventional methods of blending, filling, tabletting or the like. Repeated blending operations may be used to distribute the active agent throughout those compositions employing large quantities of fillers. Such operations are, of course, conventional in the art.

Oral liquid preparations may be in the form of, for example, aqueous or oily suspensions, solutions, emulsions, syrups, or elixirs, or may be presented as a dry product for reconstitution with water or other suitable vehicle before use. Such liquid preparations may contain conventional additives such-as suspending agents, for example sorbitol, syrup, methyl cellulose, gelatin, hydroxyethylcellulose, carboxymethyl cellulose, aluminium stearate gel or hydrogenated edible fats, emulsifying agents, for example lecithin, sorbitan monooleate, or acacia; non-aqueous vehicles (which may include edible oils), for example, almond oil, fractionated coconut oil, oily esters such as esters of glycerine, propylene glycol, or ethyl alcohol; preservatives, for example methyl or propyl *p-*hydroxybenzoate or sorbic acid, and if desired conventional flavouring or colouring agents.

Oral formulations also include conventional sustained release formulations, such as tablets or granules having an enteric coating.

For parenteral administration, fluid unit dose forms are prepared containing the compound and a sterile vehicle. The compound, depending on the vehicle and the concentration, can be either suspended or dissolved. Parenteral solutions are normally prepared by dissolving the compound in a vehicle and filter sterilising before filling into a suitable vial or ampoule and sealing. Advantageously, adjuvants such as a local anaesthetic, preservatives and buffering agents are also dissolved in the vehicle. To enhance the stability, the composition can be frozen after filling into the vial and the water removed under vacuum.

Parenteral suspensions are prepared in substantially the same manner except that the compound is suspended in the vehicle instead of being dissolved and sterilised by exposure to ethylene oxide before suspending in the sterile vehicle. Advantageously, a surfactant or wetting agent is included in the composition to facilitate uniform distribution of the compound of the invention. As is common practice, the compositions will usually be accompanied by written or printed directions for use in the medical treatment concerned.

The present invention further provides a pharmaceutical composition for use in the treatment and/or prophylaxis of degenerative diseases such as Huntingdon's chorea, schizophrenia, OCD, neurological deficits associated with AIDS, sleep disorders (including circadian rhythm disorders, insomnia and narcolepsy), tics (e.g. Giles de la Tourette's syndrome), traumatic brain injury, tinnitus, neuralgia, especially trigeminal neuralgia, neuropathic pain, dental pain, cancer pain, inappropriate neuronal activity resulting in neurodysthesias in diseases such as diabetes, MS and motor neurone disease, ataxias, muscular rigidity (spasticity) and temporomandibular joint dysfunction, which comprises a compound of formula (A), or a pharmaceutically acceptable salt or solvate thereof, and a pharmaceutically acceptable carrier.

In a further aspect the invention provides the use of a compound of formula (A), or a pharmaceutically acceptable salt or solvate thereof, for the manufacture of a medicament for the treatment and/or prophylaxis of degenerative diseases such as Huntingdon's chorea, schizophrenia, neurological deficits associated with AIDS, sleep disorders (including circadian rhythm disorders, insomnia and narcolepsy), tics (e.g. Giles de la Tourette's syndrome), traumatic brain injury, tinnitus, neuralgia, especially trigeminal neuralgia, neuropathic pain, dental pain, cancer pain, inappropriate neuronal activity resulting in neurodysthesias in diseases such as diabetes, MS and motor neurone disease, ataxias, muscular rigidity (spasticity) and temporomandibular joint dysfunction.

Such compositions and medicaments may be prepared in the manner as hereinbefore described.

This invention is particularly concerned with the treatment of neuropathic pain and trigeminal neuralgia, espesially by use of *trans*-6-acetyl-4S-(4-fluorobenzoylamino)-3,4-dihydro-2,2-dimethyl-2H-1-benzopyran-3R-ol (Compound 1) and *cis*-6-acetyl-4S-(3-chloro-4-fluorobenzoylamino)-3,4-dihydro-2,2-dimethyl-2H-1-benzopyran-3S-ol (Compound 2) and *trans*-6-acetyl-4S-(3,5-difluorobenzoylamino)-3,4-dihydro-2,2-dimethyl-2H-1-benzopyran-3R-ol (Compound 3).

The present invention is illustrated by the following pharmocological data:

### Trigeminal nerve model in anaesthetised cats

Cats were anaesthetised with α-chloralose (90-110mg/kg i.v.) and artificially respired with room air. Body temperature was maintained at 37-38 C. A femoral artery was cannulated for recording of blood pressure and heart rate. Arterial blood flow was recorded by a Doppler flow probe placed around the right common carotid artery. Bipolar stainless electrodes were stereotaxically placed into each trigeminal ganglion. Guanethidine (3 mg/kg i.v.) was then administered and 45 min allowed for stabilisation. Stimulation (2mA, 10Hz for 2 min) of the trigeminal ganglion, ipsilateral to the carotid artery from which blood flow was measured, increased blood flow and reduced carotid vascular resistance. The ability of drugs, given i.v. to modulate this response was used to assess their effects on the trigemino-vascular system. Intravenous administration of Compound 1 (n=3) or Compound 2 (n=4) at a rate of 3.4 µmol/h produced a significant inhibition of TGN-induced reduction in carotid vascular resistance at 4 hours (Table 1).

Intraduodenal administration of Compound 1 (n=3) or Compound 2 (10mg.kg) also produced significant inhibition of TGN-induced reduction in carotid vascular resistance (Table 2) after 3 hours.

**Table 1:**

| **Effects of continuous intravenous administration Compound 1 and Compound 2 on TGN-induced reduction in carotid vascular resistance in the anaesthetised cat** | | |
|---|---|---|
| | **n** | **% Change in TGN-reduction in carotid vascular resistance at 4 hours following continuous iv infusion (mean** ± **sem)** |
| control (3.4µmol/h) | 4 | 11.6 ± 8.6 |
| control (11 µmol/h) | 3 | 15.7 ± 10 |
| Compound 1 3.4µmol/h | 3 | -29.1 ± 3.7* |
| Compound 2 3.4µmol/h | 4 | -30.0 ± 6.6* |
| Compound 2 11 µmol/h | 4 | -21.8 ± 14* |

| | | |
|---|---|---|
| *P<0.05 | | |

**Table 2:**

| **Effects of intraduodenal administration of Compound 1 or 2 (10mg/kg) on TGN-induced administration in the anaethetised cat** | | |
|---|---|---|
| | **n** | % Changes of TGW-induced reduction in carotid vascular resistance 3 hours (mean ± sem) |
| control (labrosol) | 3 | 30.6 ± 12.2 |
| Compound 1 (labrasol) | 3 | -43 ± 12.9* |
| control (methylcellulose) | 2 | 6.8 |
| Compound 2 (methylcellulose) | 2 | -69 |

| | | |
|---|---|---|
| *P<0.05 | | |

Compound 3 had marked effects on TGN stimulation-induced reductions in carotid vascular resistance in the guanethidine treated anaesthetised cat. In the absence of drug treatment, TGN stimulation typically produced an approximate 40-50% reduction in carotid vascular resistance which was reproducible at 30 minute intervals. At 2 hours post administration, intraduodenal bolus administration of Compound 3 (10 mg/kg) produced a 77% (n=4) inhibition of trigeminal nerve mediated responses, whereas no inhibition was observed in vehicle treated animals (-4.1%, n=3).

### Neuropathic Pain Model in Anaesthetised Rats.

The left sciatic nerve was exposed in anaesthetised rats and dorsal 50-60% of the nerve ligated as described by Seltzer et al 1990 (Pain 43, p205-218). The surgical wound was then closed and the animals allowed to recover. Thermal nociception was measured by latency to withdraw hind paw from an infrared light source pre-surgery (8 days and 1 day before surgery) and at regular intervals following nerve ligation.

Compounds were administered as a single or repeat dose on established hyperalgesia, typically 14 days post ligation. Compounds were administered as either an oral or systemic formulation and the change in thermal nociception recorded for up to 40 days.

## Claims

1. Use in the manufacture of a medicament for use in the treatment and/or prophylaxis of neuralgia, especially trigeminal neuralgia, neuropathic pain, dental pain, and cancer pain, of a compound of formula (A), or a pharmaceutically acceptable salt or solvate thereof: 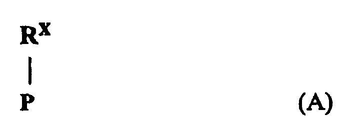 wherein:
P is a ring system: 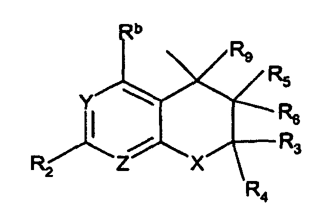 wherein:
either Y is N and R₂ is hydrogen, or Y is C-R₁; where:
either one of R₁ and R₂ is hydrogen and the other is selected from the class of hydrogen, C₃₋₈ cycloalkyl, C₁₋₆ alkyl optionally interrupted by oxygen or substituted
by hydroxy, C₁₋₆ alkoxy or substituted aminocarbonyl, C₁₋₆ alkylcarbonyl, C₁₋₆ alkoxycarbonyl, C₁₋₆ alkylcarbonyloxy, C₁₋₆ alkoxy, nitro, cyano, halo,
trifluoromethyl, CF₃S, or a group CF₃-A-, where A is -CF₂-, -CO-, -CH₂-, CH(OH), SO₂, SO, CH₂-O, or CONH, or a group CF₂H-A'- where A' is oxygen, sulfur, SO, SO₂, CF₂ or CFH; trifluoromethoxy, C₁₋₆ alkylsulfinyl, perfluoro C₂₋₆ alkylsulfonyl, C₁₋₆ alkylsulfonyl, C₁₋₆ alkoxysulfinyl, C₁₋₆ alkoxysulfonyl, aryl, heteroaryl, arylcarbonyl, heteroarylcarbonyl, phosphono, arylcarbonyloxy, heteroarylcarbonyloxy, arylsulfinyl, heteroarylsulfinyl, arylsulfonyl, heteroarylsulfonyl in which any aromatic moiety is optionally substituted, C₁₋₆ alkylcarbonylamino, C₁₋₆ alkoxycarbonylamino, C₁₋₆ alkyl-thiocarbonyl, C₁₋₆ alkoxy-thiocarbonyl, C₁₋₆ alkyl-thiocarbonyloxy, 1-mercapto C₂₋₇ alkyl, formyl, or aminosulfinyl, aminasulfonyl or aminocarbonyl, any amino moiety being optionally substituted by one or two C₁₋₆ alkyl groups, or C₁₋₆ alkylsulfinylamino, C₁₋₆ alkylsulfonylamino,C₁₋₆ alkoxysulfinylamino or C₁₋₆ alkoxysulfonylamino, or ethylenyl terminally substituted by C₁₋₆ alkylcarbonyl, nitro or cyano, or -C(C₁₋₆ alkyl)NOH or -C(C₁₋₆ alkyl)NNH₂,
or one of R₁ and R₂ is nitro, cyano or C₁₋₃ alkylcarbonyl and the other is halo, C₁₋₄ alkyl, methoxy or amino optionally substituted by one or two C₁₋₆ alkyl or by C₂₋₇ alkanoyl;
or R₁ and R₂ together are -(CH₂)₄-; (CH₂)ₓ CO (CH₂)y where x is 0 to 3 and y is 0 to 3 with the proviso that x + y is at least 2x; or -CH = CH-CH = CH-; or form an optionally substituted triazole or oxadiazole ring, or together form a group CONR^{c}CO where R^{c} is hydrogen, C₁₋₆ alkyl, aralkyl or heteroarylalkyl;
Z is N only when Y is C-R₁ or Z is C-R^{a} when Y is N or C-R₁; wherein R^{a} is hydrogen, halogen, nitro; C₁₋₄ alkylcarbonyl, C₁₋₄ alkyl; aryl C₁₋₄ alkyl, aryl C₁₋₄ alkenyl, heteroaryl C₁₋₄ alkyl or heteroaryl C₁₋₄ alkenyl,
R^{b} is hydrogen, halogen, nitro; C₁₋₄ alkylcarbonyl or C₁₋₄ alkyl; and in which any aryl or heteroaryl or alkyl moeity associated with R^{a} or R^{b} are optionally substituted;
one of R₃ and R₄ is hydrogen or C₁₋₄ alkyl and the other is C₁₋₄ alkyl, CF₃
or CH₂X^{a} where X^{a} is fluoro, chloro, bromo, iodo, C₁₋₄ alkoxy, hydroxy, C₁₋₄ alkylcarbonyloxy, -S-C₁₋₄ alkyl, nitro, amino optionally substituted by one or two C₁₋₄ alkyl groups; cyano or C₁₋₄ alkoxycarbonyl
or R₃ and R₄ together are C₂₋₅ polymethylene optionally substituted by C₁₋₄ alkyl;
R₅ is C₁₋₆ alkylcarbonyloxy, benzoyloxy, ONO₂, benzyloxy, phenyloxy or C₁₋₆ alkoxy and R₆ and R₉ are hydrogen or R₅ is hydroxy and R₆ and R₉ are independently hydrogen or C₁₋₂ alkyl;
R^{x} is (a R₈-N-CO-R₇ group where R₇ is heteroaryl or phenyl; both of which are optionally substituted one or more times independently with a group or atom selected from chloro, fluoro, bromo, iodo, nitro, amino optionally substituted once or twice by C₁₋₄ alkyl, cyano, azido, C₁₋₄ alkyl, C₁₋₄ alkoxy, trifluoromethoxy, trifluoromethyl; optionally substituted aryloxy or heteroaryloxy; C₁₋₄ alkoxy substituted by one or more halogens (excluding trifluoromethoxy); amino substituted by C₁₋₄ alkanoyl, aroyl aryl phenylsulfonyl or C₁₋₄ alkylsulfonyl; C₁₋₄ alkyl substituted by one or more halogens (excluding trifluoromethyl) or alkoxy; phenylsulfonyl C₁₋₄ alkyl sulfonyl, aminosulfonyl in which the amino group is optionally substituted by C₁₋₄ alkyl; CONH₂ in which the amino group is optionally substituted by C₁₋₄ alkyl;
R₈ is hydrogen; C₁₋₆ alkyl, OR₁₆ or NHCOR₁₇ wherein R₁₆ is hydrogen, C₁₋₆ alkyl, formyl, C₁₋₆ alkanoyl, aroyl or aryl-C₁₋₆ alkyl and R₁₇ is hydrogen, C₁₋₆ alkyl, C₁₋₆ alkoxy, mono or di C₁₋₆ alkyl amino, amino, amino-C₁₋₆ alkyl, hydroxy-C₁₋₆ alkyl, halo-C₁₋₆ alkyl, C₁₋₆ acyloxy-C₁₋₆ alkyl, C₁₋₆ alkoxycarbonyl-C₁₋₆-alkyl, aryl or heteroaryl;
X is oxygen or NR₁₈ where R₁₈ is hydrogen or C₁₋₆ alkyl; and the R^{x} group is *cis* or *trans* to the R₅ group.

2. Use according to claim 1, for the treatment of trigeminal neuralgia.

3. Use according to claim 1, for the treatment of neuropathic pain.

4. Use according according to claim 1, in which the compound of formula (A) is *trans*-6-acetyl-4S-(4-fluorobenzoylamino)-3,4-dihydro-2,2-dimethyl-2H-1-benzopyran-3R-ol or *cis*-6-acetyl-4S-(3-chloro-4-fluorobenzoylamino)-3,4-dihydro-2,2-dimethyl-2H-1-benzopyran-3S-ol or *trans*-6-acetyl-4S-(3,5-difluorobenzoyl-amino)-3,4-dihydro-2,2-dimethyl-2H-1-benzopyran-3R-ol, or a pharmaceutically acceptable salt or solvate thereof.

## Patentansprüche

1. Verwendung einer Verbindung der Formel (A) 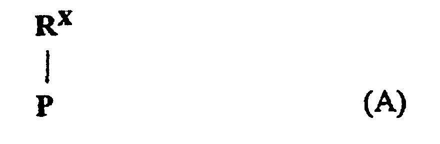 wobei
P ein Ringsystem ist: 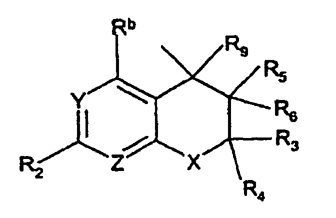 wobei
entweder Y gleich N ist und der Rest R₂ Wasserstoff ist oder Y die Bedeutung C-R₁ hat; wobei
entweder einer der Reste R₁ und R₂ Wasserstoff ist und der andere ausgewählt ist aus Wasserstoff, C₃₋₈-Cycloalkyl, C₁₋₆-Alkyl, gegebenenfalls unterbrochen mit Sauerstoff oder substituiert mit Hydroxy, C₁₋₆-Alkoxy oder substituiertem Aminocarbonyl, C₁₋₆-Alkylcarbonyl, C₁₋₆-Alkoxycarbonyl, C₁₋₆-Alkylcarbonyloxy, C₁₋₆-Alkoxy, Nitro, Cyano, Halogen, Trifluormethyl, CF₃S, oder einer Gruppe CF₃-A-, wobei A die Bedeutung -CF₂-, -CO-, -CH₂-, CH(OH), SO₂, SO, CH₂-O, oder CONH hat, oder einer Gruppe CF₂H-A'-, wobei A' Sauerstoff, Schwefel, SO, SO₂, CF₂ oder CFH ist; Trifluormethoxy, C₁₋₆-Alkylsulfinyl, Perfluor-C₂₋₆-alkylsulfonyl, C₁₋₆-Alkylsulfonyl, C₁₋₆-Alkoxysulfinyl, C₁₋₆-Alkoxysulfonyl, Aryl, Heteroaryl, Arylcarbonyl, Heteroarylcarbonyl, Phosphono, Arylcarbonyloxy, Heteroarylcarbonyloxy, Arylsulfinyl, Heteroarylsulfinyl, Arylsulfonyl, Heteroarylsulfonyl, wobei jede aromatische Einheit gegebenenfalls substituiert ist, C₁₋₆-Alkylcarbonylamino, C₁₋₆-Alkoxycarbonylamino, C₁₋₆-Alkylthiocarbonyl, C₁₋₆-Alkoxythiocarbonyl, C₁₋₆-Alkylthiocarbonyloxy, 1-Mercapto-C₂₋₇-alkyl, Formyl, oder Aminosulfinyl, Aminosulfonyl oder Aminocarbonyl, wobei jede Aminoeinheit gegebenenfalls mit einem oder zwei C₁₋₆-Alkylresten substituiert ist, oder C₁₋₆-Alkylsulfinylamino, C₁₋₆-Alkylsulfonylamino, C₁₋₆-Alkoxysulfinylamino oder C₁₋₆-Alkoxysulfonylamino, oder Ethylenyl, das endständig substituiert ist mit C₁₋₆-Alkylcarbonyl, Nitro oder Cyano, oder -C(C₁₋₆-Alkyl)NOH oder -C(C₁₋₆-Alkyl)NNH₂;
oder einer der Reste R₁ und R₂ Nitro, Cyano oder C₁₋₃-Alkylcarbonyl ist und der andere Halogen, C₁₋₄-Alkyl, Methoxy oder Amino, das gegebenenfalls mit einem oder zwei C₁₋₆-Alkyl oder mit C₂₋₇-Alkanoyl substituiert ist, ist;
oder die Reste R₁ und R₂ zusammen -(CH₂)₄-, (CH₂)ₓCO(CH₂)_{y}, wobei x die Bedeutung 0 bis 3 hat und y die Bedeutung 0 bis 3 hat, mit der Maßgabe, daß x + y mindestens 2x ist, oder -CH=CH-CH=CH- bilden; oder einen gegebenenfalls substituierten Triazol- oder Oxadiazolring bilden, oder zusammen eine Gruppe CONR^{c}CO bilden, wobei der Rest R^{c} Wasserstoff, C₁₋₆-Alkyl, Aralkyl oder Heteroarylalkyl ist;
Z nur N ist, wenn Y die Bedeutung C-R₁ hat, oder Z die Bedeutung C-R^{a} hat, wenn Y gleich N oder C-R₁ ist; wobei der Rest R^{a} Wasserstoff, Halogen, Nitro, C₁₋₄-Alkylcarbonyl, C₁₋₄-Alkyl, Aryl-C₁₋₄-alkyl, Aryl-C₁₋₄-alkenyl, Heteroaryl-C₁₋₄-alkyl oder Heteroaryl-C₁₋₄-alkenyl ist,
der Rest R^{b} Wasserstoff, Halogen, Nitro, C₁₋₄-Alkylcarbonyl oder C₁₋₄-Alkyl ist und wobei jede Aryl- oder Heteroaryl- oder Alkyleinheit, die mit den Resten R^{a} oder R^{b} verbunden ist, gegebenenfalls substituiert ist;
einer der Reste R₃ und R₄ Wasserstoff oder C₁₋₄-Alkyl ist und der andere C₁₋₄-Alkyl, CF₃ oder CH₂X^{a}, wobei X^{a} Fluor, Chlor, Brom, Iod, C₁₋₄-Alkoxy, Hydroxy, C₁₋₄-Alkylcarbonyloxy, -S-C₁₋₄-Alkyl, Nitro, Amino, das gegebenenfalls mit einer oder zwei C₁₋₄-Alkylresten substituiert ist, ist, Cyano oder C₁₋₄-Alkoxycarbonyl ist;
oder die Reste R₃ und R₄ zusammen C₂₋₅-Polymethylen bilden, das gegebenenfalls mit C₁₋₄-Alkyl substituiert ist;
der Rest R₅ die Bedeutung C₁₋₆-Alkylcarbonyloxy, Benzoyloxy, ONO₂, Benzyloxy, Phenyloxy oder C₁₋₆-Alkoxy hat und die Reste R₆ und R₉ Wasserstoff sind oder der Rest R₅ Hydroxy ist und die Reste R₆ und R₉ unabhängig Wasserstoff oder C₁₋₂-Alkyl sind;
der Rest R^{x} ein R₈-N-CO-R₇-Rest ist, wobei der Rest R₇ Heteroaryl oder Phenyl ist, von denen beide gegebenenfalls ein- oder mehrmals unabhängig mit einem Rest oder Atom substituiert sind, ausgewählt aus Chlor, Fluor, Brom, Iod, Nitro, Amino, gegebenenfalls einmal oder zweimal substituiert mit C₁₋₄-Alkyl, Cyano, Azido, C₁₋₄-Alkyl, C₁₋₄-Alkoxy, Trifluormethoxy, Trifluormethyl; gegebenenfalls substituiertem Aryloxy oder Heteroaryloxy; C₁₋₄-Alkoxy, das mit einem oder mehreren Halogenen (ausgeschlossen Trifluormethoxy) substituiert ist; Amino, das mit C₁₋₄-Alkanoyl, Aroylarylphenylsulfonyl oder C₁₋₄-Alkylsulfonyl substituiert ist; C₁₋₄-Alkyl, das mit einem oder mehreren Halogenen (ausgeschlossen Trifluormethyl) oder Alkoxy substituiert ist; Phenylsulfonyl-C₁₋₄-alkylsulfonyl, Aminosulfonyl, wobei die Aminogruppe gegebenenfalls mit C₁₋₄-Alkyl substituiert ist; CONH₂, wobei die Aminogruppe gegebenenfalls mit C₁₋₄-Alkyl substituiert ist;
der Rest R⁸ Wasserstoff, C₁₋₆-Alkyl, OR₁₆ oder NHCOR₁₇ ist, wobei der Rest R₁₆ Wasserstoff, C₁₋₆-Alkyl, Formyl, C₁₋₆-Alkanoyl, Aroyl oder Aryl-C₁₋₆-alkyl ist und der Rest R₁₇ Wasserstoff, C₁₋₆-Alkyl, C₁₋₆-Alkoxy, mono- oder di-C₁₋₆-Alkylamino, Amino, Amino-C₁₋₆-alkyl, Hydroxy-C₁₋₆-alkyl, Halogen-C₁₋₆-alkyl, C₁₋₆-Acyloxy-C₁₋₆-alkyl, C₁₋₆-Alkoxycarbonyl-C₁₋₆-alkyl, Aryl oder Heteroaryl ist;
X Sauerstoff oder NR₁₈ ist, wobei der Rest R₁₈ Wasserstoff oder C₁₋₆-Alkyl ist; und der R^{x}-Rest cis oder trans zu dem R₅-Rest ist;
oder eines pharmazeutisch verträglichen Salzes oder Solvats davon zur Herstellung eines Medikaments zur Verwendung in der Behandlung und/oder Prophylaxe von Neuralgie, insbesondere Trigeminusneuralgie, neuropathischem Schmerz, Zahnschmerz und Krebsschmerz.

2. Verwendung gemäß Anspruch 1 zur Behandlung von Trigeminusneuralgie.

3. Verwendung gemäß Anspruch 1 zur Behandlung von neuropathischem Schmerz.

4. Verwendung gemäß Anspruch 1, wobei die Verbindung der Formel (A) trans-6-Acetyl-4S-(4-fluorbenzoylamino)-3,4-dihydro-2,2-dimethyl-2H-1-benzopyran-3R-ol oder
cis-6-Acetyl-4S-(3-chlor-4-fluorbenzoylamino)-3,4-dihydro-2,2-dimethyl-2H-1-benzopyran-3S-ol oder *trans*-6-Acetyl-4S-(3,5-difluorbenzoylamino)-3,4-dihydro-2,2-dimethyl-2H-1-benzopyran-3R-ol oder ein pharmazeutisch verträgliches Salz oder Solvat davon ist.

## Revendications

1. Utilisation dans la production d'un médicament destiné à être utilisé dans le traitement et/ou la prophylaxie des névralgies, notamment des névralgies du trijumeau, d'une douleur neuropathique, d'une douleur dentaire, et d'une douleur provoquée par un cancer, d'un composé de formule (A), ou d'un ses sels ou produits de solvatation pharmaceutiquement acceptables : 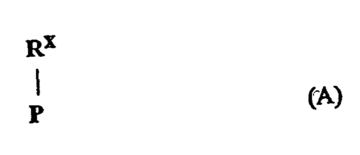 formule dans laquelle :
P représente un système cyclique : 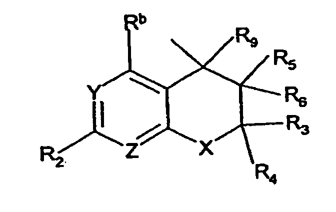
dans lequel :
Y représente un atome de N et R₂ représente un atome d'hydrogène, ou bien Y représente un groupe C-R₁ ; où :
un des groupes R₁ et R₂ représente un atome d'hydrogène et l'autre est choisi dans le groupe consistant en un atome d'hydrogène, des groupes cycloalkyle en C₃ à C₈, alkyle en C₁ à C₆ facultativement interrompu par un atome d'oxygène ou substitué avec un substituant hydroxy, alkoxy en C₁ à C₆ ou aminocarbonyle substitué, (alkyle en C₁ à C₆) carbonyle, (alkoxy en C₁ à C₆) carbonyle, (alkyle en C₁ à C₆) carbonyloxy, alkoxy en C₁ à C₆, nitro, cyano, halogène, trifluorométhyle, CF₃S, ou un groupe CF₃-A-, dans lequel A représente un groupe -CF₂-, -CO-, CH₂-, CH (OH) , SO₂, SO, CH₂-O, ou CONH, ou un groupe CF₂H-A'- dans lequel A' représente un atome d'oxygène ou de soufre, un groupe SO, SO₂, CF₂ ou CFH ; trifluorométhoxy, alkylsulfinyle en C₁ à C₆, perfluoralkylsulfonyle en C₂ à C₆, alkylsulfonyle en C₁ à C₆, alkoxysulfinyle en C₁ à C₆, alkoxysulfonyle en C₁ à C₆, aryle, hétéroaryle, arylcarbonyle, hétéroarylcarbonyle, phosphono, arylcarbonyloxy, hétéroarylcarbonyloxy, arylsulfinyle, hétéroarylsulfinyle, arylsulfonyle, hétéroarylsulfonyle dans lequel n'importe quel groupement aromatique est facultativement substitué, (alkyle en C₁ à C₆) carbonylamino, (alkoxy en C₁ à C₆) carbonylamino, (alkyle en C₁ à C₆) thiocarbonyle, (alkoxy en C₁ à C₆) thiocarbonyle, (alkyle en C₁ à C₆) thiocarbonyloxy, 1-mercapto (alkyle en C₂ à C₇), formyle ou aminosulfinyle, aminosulfonyle ou aminocarbonyle, n'importe quel groupement amino étant facultativement substitué avec un ou deux groupes alkyle en C₁ à C₆, ou alkylsulfinylamino en C₁ à C₆, alkylsulfonylamino en C₁ à C₆, alkoxysulfinylamino en C₁ à C₆ ou alkoxysulfonylamino en C₁ à C₆, ou éthylényle à substitution terminale avec un substituant (alkyle en C₁ à C₆) carbonyle, nitro ou cyano, ou -C(alkyle en C₁ à C₆)NOH ou -C(alkyle en C₁ à C₆)NNH₂,
ou bien un des groupes R₁ et R₂ représente un groupe nitro, cyano ou (alkyle en C₁ à C₃) carbonyle et l'autre représente un groupe halogéno, alkyle en C₁ à C₄, méthoxy ou amino facultativement substitué avec un ou deux substituants alkyle en C₁ à C₆ ou avec un substituant alcanoyle en C₂ à C₇ ;
ou bien R₁ et R₂, conjointement, représentent un groupe -(CH₂)₄- ; (CH₂)ₓCO(CH₂)_{y} dans lequel x a une valeur de 0 à 3 et y a une valeur de 0 à 3, sous réserve que la somme x + y soit égale à au moins 2x ; ou -CH=CH-CH=CH- ; ou forment un noyau triazole ou oxadiazole facultativement substitué ou bien, conjointement, forment un groupe CONR^{c}CO dans lequel R^{c} représente un atome d'hydrogène, un groupe alkyle en C₁ à C₆, aralkyle ou hétéroarylalkyle ;
Z représente un atome de N seulement lorsque Y représente un groupe C-R₁ ou bien Z représente un groupe C-R^{a} lorsque Y représente un atome de N ou un groupe C-R₁ ; R^{a} représente un atome d'hydrogène, un atome d'halogène, un groupe nitro ; un groupe (alkyle en C₁ à C₄) carbonyle, alkyle en C₁ à C₄ ; aryl (alkyle en C₁ à C₄), aryl (alcényle en C₁ à C₄), hétéroaryl (alkyle en C₁ à C₄) ou hétéroaryl (alcényle en C₁ à C₄),
R^{b} représente un atome d'hydrogène, un atome d'halogène, un groupe nitro ; un groupe (alkyle en C₁ à C₄) carbonyle ou alkyle en C₁ à C₄ ; n'importe quel groupement aryle ou hétéroaryle ou alkyle associé à R^{a} ou R^{b} étant facultativement substitué ;
un des groupes R₃ et R₄ représente un atome d'hydrogène ou un groupe alkyle en C₁ à C₄ et l'autre représente un groupe alkyle en C₁ à C₄, CF₃ ou CH₂X^{a} dans lequel X^{a} représente un groupe fluoro, chloro, bromo, iodo, alkoxy en C₁ à C₄, hydroxy, (alkyle en C₁ à C₄) carbonyloxy, -S-(alkyle en C₁ à C₄), nitro, amino facultativement substitué avec un ou deux groupes alkyle en C₁ à C₄ ; cyano ou (alkoxy en C₁ à C₄) carbonyle
ou bien R₃ et R₄, conjointement, représentent un groupe polyméthylène en C₂ à C₅ facultativement substitué avec un substituant alkyle en C₁ à C₄ ;
R₅ représente un groupe (alkyle en C₁ à C₆) carbonyloxy, benzoyloxy, ONO₂, benzyloxy, phényloxy ou alkoxy en C₁ à C₆ et R₆ et R₉ représentent des atomes d'hydrogène, ou bien R₅ représente un groupe hydroxy et R₆ et R₉ représentent indépendamment des atomes d'hydrogène ou des groupes alkyle en C₁ ou C₂ ;
R^{x} représente un groupe R₈-N-CO-R₇ dans lequel R₇ représente un groupe hétéroaryle ou phényle ; ces deux groupes étant facultativement susbtitués une ou plusieurs fois indépendamment avec un groupe ou atome choisi entre chloro, fluoro, bromo, iodo, nitro, amino facultativement substitué une ou deux fois avec un substituant alkyle en C₁ à C₄, cyano, azido, alkyle en C₁ à C₄, alkoxy en C₁ à C₄, trifluorométhoxy, trifluorométhyle ; aryloxy ou hétéroaryloxy facultativement substitué ; alkoxy en C₁ à C₄ substitué avec un ou plusieurs atomes d'halogènes (à l'exclusion de trifluorométhoxy) ; amino substitué avec un substituant alcanoyle en C₁ à C₄, aroylarylphénylsulfonyle ou alkylsulfonyle en C₁ à C₄ ; alkyle en C₁ à C₄ substitué avec un ou plusieurs atomes d'halogènes (à l'exclusion de trifluorométhyle) ou alkoxy ; phénylsulfonyl (alkyle en C₁ à C₄) sulfonyle, aminosulfonyle dans lequel le groupe amino est facultativement substitué avec un substituant alkyle en C₁ à C₄ ; CONH₂ dans lequel le groupe amino est facultativement substitué avec un substituant alkyle en C₁ à C₄ ;
R₈ représente un atome d'hydrogène ; un groupe alkyle en C₁ à C₆, OR₁₆ ou NHCOR₁₇ dans lequel R₁₆ représente un atome d'hydrogène, un groupe alkyle en C₁ à C₆, formyle, alcanoyle en C₁ à C₆, aroyle ou aryl (alkyle en C₁ à C₆) et R₁₇ représente un atome d'hydrogène, un groupe alkyle en C₁ à C₆, alkoxy en C₁ à C₆, mono- ou di (alkyle en C₁ à C₆)-amino, amino, amino-alkyle en C₁ à C₆, hydroxy(alkyle en C₁ à C₆), halogéno (alkyle en C₁ à C₆), (acyle en C₁ à C₆)-oxy-(alkyle en C₁ à C₆), (alkoxy en C₁ à C₆) carbonyl (alkyle en C₁ à C₆) aryle ou hétéroaryle ;
X représente un atome d'oxygène ou un groupe NR₁₈ dans lequel R₁₈ représente un atome d'hydrogène ou un groupe alkyle en C₁ à C₆ ; et le groupe R^{x} est en position *cis* ou *trans* par rapport au groupe R₅.

2. Utilisation suivant la revendication 1, pour le traitement des névralgies du trijumeau.

3. Utilisation suivant la revendication 1, pour le traitement d'une douleur neuropathique.

4. Utilisation suivant la revendication 1, dans laquelle le composé de formule (A) est le trans-6-acétyl-4S-(4-fluorobenzoylamino)-3,4-dihydro-2,2-diméthyl-2H-1-benzopyranne-3R-ol ou le *cis*-6-acétyl-4S-(3-chloro-4-fluorobenzoylamino)-3,4-dihydro-2,2-diméthyl-2H-1-benzopyranne-3S-ol ou le *trans*-6-acétyl-4S-(3,5-difluorobenzoylamino)-3,4-dihydro-2,2-diméthyl-2H-1-benzopyranne-3R-ol, ou un de leurs sels ou produits de solvatation pharmaceutiquement acceptables.
